# EUROPEAN PATENT APPLICATION

(11) **EP 4 151 246 A1**
(43) Date of publication of application: **22.03.2023**
(21) Application number: 22818601.1
(22) Date of filing: 14.06.2022
(51) Int. Cl.: A61L 31/04, A61K 31/728, A61K 31/734, A61L 15/16, A61L 31/00, A61L 31/08, A61L 31/14, A61P 31/04, A61P 35/00, C07C 69/587

(54) **ANTI-ADHESION COMPOSITION**

(30) Priority: 14.06.2021 KR 20210076956
(71) Applicant: CNLD CO., LTD., Seoul 06373 (KR); UNIVERSITY-INDUSTRY FOUNDATION (UIF), YONSEI UNIVERSITY, Seoul 03722 (KR)
(72) Inventor: HWANG, Sung Joo, Seoul 06370 (KR); KIM, Dong Min, Hanam-si Gyeonggi-do 12915 (KR); KIM, Ji Eun, Incheon 21997 (KR); CHOI, Yeon Ju, Suwon-si Gyeonggi-do 16634 (KR); HWANG, Yong Kyung, Seoul 03004 (KR)
(74) Representative: Moser Götze & Partner Patentanwälte mbB
(86) International application number: PCT/KR2022/008430
(87) International publication number: WO 2022/265367

(57) **Abstract**

Disclosed is an anti-adhesion agent composition which uses a high molecular weight compound having biocompatibility and an oil derived from animals, plants, and minerals so as to improve low viscosity and low strength, which are problems of existing anti-adhesion agents, and prevent adhesion between living tissues. The anti-adhesion composition according to the present disclosure performs a physical barrier function through simple mixing of a polymer material, serving as a physical barrier in its anti-adhesion effect, and a solvent, and remains in the body for a certain period of time, then decomposes, absorbs, and is excreted. The anti-adhesion composition does not interfere with the healing of the wound after surgery, and exhibits an excellent effect of convenient application to the surgical site.

## Description

### [Technical Field]

The present disclosure relates to an anti-adhesion agent composition and a method for manufacturing the same, and more particularly, to an anti-adhesion composition for preventing adhesion of tissue from occurring at a surgical site and a method for manufacturing the same.

### [Background Art]

Adhesion, which generally occurs after surgery in the process of recovery from inflammation, wounds, *etc.,* refers to the adhesion of organs and tissues around the wound site. In detail, an inflammatory reaction and a coagulation cascade occur in the wound healing process to generate fibrins. Excessive secretion of the amount of produced fibrins causes an increase in fibroblasts and forms new blood vessels, resulting in adhesion between tissues. Adhesion occurs between the abdominal wall and the intestine of a patient after surgery with a high probability of 60% to 93%.

Adhesion causes serious problems. When part of the intestine is tightened due to intra-abdominal adhesions, the blood supply to the intestine is cut off, which can lead to ischemia; in addition, it can cause problems such as intestinal dysfunction, chronic pain, and infertility. To prevent such adhesions, anti-inflammatory drugs or physical barriers are currently used.

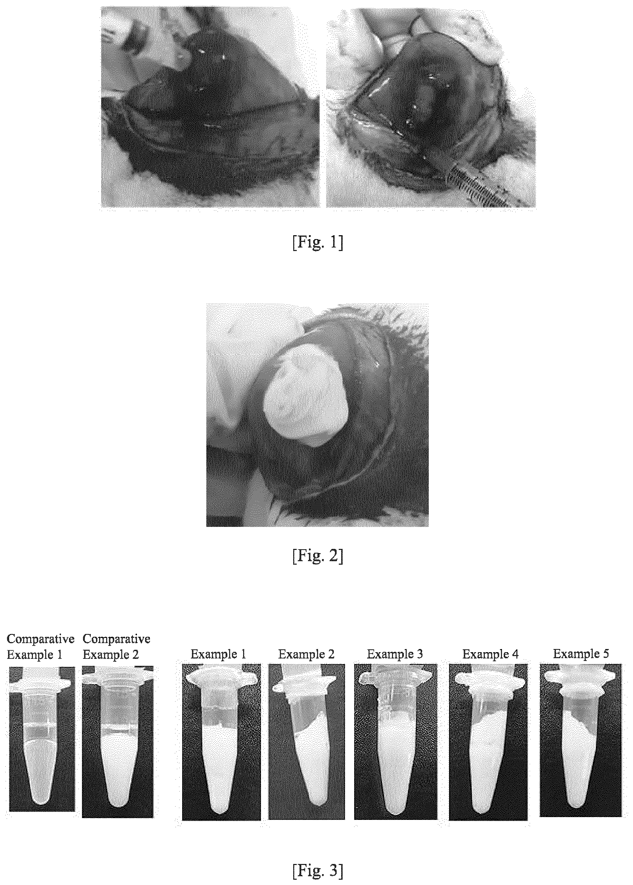

Previously invented anti-adhesion agents are sold in the form of a polymer film (in the form of a film or fabric) or in the form of a liquid or hydrogel. As a film-type anti-adhesion agent, Seprafilm^{®} (Genzyme, where hyaluronic acid is cross-linked with carboxymethyl cellulose), Interceed^{®} (Johnson & Johnson Medical, which uses oxidized regenerated cellulose (ORC)), and Surgiwrap^{®} (Mast Biosurgery, which uses polylactic acid) are commercially available. These products have problems in that they do not attach well to the surface of organs when applied to the body, the film rolls up when they are brought into contact with body fluids, they have insufficient mechanical durability in dried conditions, they clump together or depart from the applied site by organ movement after adhesion, they biodegrade very slow in the body thus requiring them to be removed by surgery, *etc.* This type of film has problems in that it is difficult to apply to a narrow area, does not attach well to the surface of organs, and clumps together by the movement of organs, thus having an insufficient anti-adhesion effect or having difficulty in its application. The anti-adhesion agent in a liquid agent or hydrogel form lacks mechanical durability as a physical barrier and has limited adhesion to the wound site (FIG. 1).

In this regard, Korean Patent No. 10-1062068 relates to an anti-adhesion composition for preventing tissue adhesion containing hyaluronic acid and hydroxyethyl starch or chitooligosaccharide, and the composition is marketed under the product name Medicurtain^{®}. However, the anti-adhesion composition used in this patent is a gel formulation, and its local application is difficult due to its insufficient adhesion at the wound site. The amount of the anti-adhesion composition used in Korean Patent No. 10-1062068 is 5 mL, which is an extremely large amount compared to the local wound site. However, it can be confirmed in "Control Example 4" of the present disclosure that when the anti-adhesion composition is used in an amount of 5 mL, which is an amount applied only to local wounds, adhesion occurs due to an insufficient anti-adhesion effect.

Although the oil used in the present disclosure is known as a component that inhibits adhesions, it cannot stay well in the wound site and thus cannot exhibit a sufficient anti-adhesion effect. However, if a water-soluble polymer and oil are properly emulsified, the oil can stay in the wound for a long period of time and effectively inhibit adhesions.

The hydrophilic polymer used in the present disclosure is a kind of thickener that increases viscosity and is a pharmaceutically acceptable excipient. Due to the excellent biocompatibility, the hydrophilic polymer is widely used in pharmaceutical products including syrups, suspensions, tablets, *etc.*

As such, in the present disclosure, as a result of studying an effective anti-adhesion agent while supplementing adhesion to the wound site and mechanical durability, which are disadvantages of various types of anti-adhesion agents invented previously, an anti-adhesion agent of a novel formulation was completed in which a hydrophilic polymer and oils derived from animals, plants, or minerals were used. (FIG. 2).

### [Disclosure]

### [Technical Problem]

An object of the present disclosure is to provide a composition for preventing tissue adhesion, which includes a water phase component, an oil phase component including an oil derived from animals, plants, or minerals, and also includes a hydrophilic polymer, a surfactant and a co-surfactant (co-solvent), and preferably an oil-in-water type of emulsion composition. An object of the present disclosure is to effectively form a physical barrier at the surgical site by increasing physical viscoelasticity through a composition including a hydrophilic polymer and an oil derived from animals, plants, or minerals to enable sufficient retention at the surgical site during the wound healing period to thereby prevent adhesions at the surgical site. The present disclosure is a formulation in the form of an emulsion and its application is not limited to a specific surgical technique but can be used in a variety of ways.

### [Technical Solution]

The anti-adhesion composition according to the present disclosure may include hydrophilic polymers; oils derived from animals, plants or minerals; surfactants; and a co-surfactant selected from the group consisting of ethanol, glycerol, polyethylene glycol, propylene carbonate, propylene glycol, dimethylformamide (DMF), dimethyl sulfoxide (DMSO), dimethylamine (DMA), triacetin, N-methyl-2-pyrrolidone (NMP), diethylene glycol monoethyl ether, polyoxylglyceride, and a combination thereof.

In one embodiment, the oil derived from animals, plants, or minerals may be contained in a range of 1 wt% to 50 wt% based on the total weight.

In one embodiment, the hydrophilic polymer may be contained in a range of 0.1 wt% to 20 wt% based on the total weight.

In one embodiment, the surfactant may be contained in a range of 0. 1 wt% to 10 wt% based on the total weight.

In one embodiment, the co-surfactant may be contained in a range of 1 wt% to 50 wt% based on the total weight.

In one embodiment, the viscosity of the anti-adhesion agent composition may be in a range of 0 mPa s. to 300,000 mPa s.

In one embodiment, the hydrophilic polymer may be selected from the group consisting of starch, hyaluronic acid, sodium alginate, pectin, carboxymethyl cellulose sodium, methyl cellulose, hydroxypropyl methyl cellulose, low-substituted hydroxypropyl cellulose, hydroxypropyl cellulose, dextran, pullulan, polyvinylpyrrolidone, polyacrylic acid, polyvinyl alcohol, polyethylene glycol, chitosan, gelatin, carrageenan, guar gum, locust bean gum, tragacantha, xanthan gum, gellan gum, agarose, and a combination thereof.

In one embodiment, the oil derived from animals, plants, or minerals may be selected from the group consisting of squalene, lanolin, mineral oil, paraffin, vaseline, almond oil, corn oil, cottonseed oil, olive oil, peanut oil, safflower oil, grapeseed oil, sesame oil, soybean oil, argan oil, canola oil, castor oil, coconut oil, sunflower oil, palm oil, avocado oil, and a combination thereof.

In one embodiment, the surfactant may be selected from the group consisting of may be selected from the group consisting of polyoxyethylene based fatty ethers derived from lauryl, cetyl, stearyl, and oleyl alcohols, caprylocaproyl macrogol glycerides, polyethoxylated castor oil, ethyloxide/12-hydroxy stearic acid, sucrose fatty acid ester, polyoxyethylene sorbitan fatty acid ester, sorbitan ester, a block copolymer of polyoxyethylene and polyoxypropylene, sodium lauryl sulfate, sodium oleate, sodium dioctyl sulfosuccinate, quaternary ammonium salt, imidazoline salt, α-tocopherol-polyethylene glycol-1000-succinate, lecithin, and a combination thereof.

### [Advantageous Effects]

The anti-adhesion composition in the form of an emulsion formulation according to the present disclosure exhibits excellent biocompatibility, duration in the body, and tissue adhesion, thereby ultimately exhibiting an excellent anti-adhesion effect. The composition of the present disclosure is a formulation in the form of an emulsion and is not limited to a specific surgical technique but can be used in a variety of ways. In addition, the following examples are illustrative of the present disclosure, and the present disclosure is not limited to these examples.

### [Description of Drawings]

FIG. 1 shows images in which Medicacon^{®} (left) of Shin Poong Pharm. Co., Ltd. and Guardix^{®}-sol (right) of Hanmi Healthcare Inc., which are commercially available liquid-form anti-adhesion agents, are being applied to the abdominal wall of a rat.
FIG. 2 shows an image in which the anti-adhesion agent in the form of an oil-in-water emulsion of the present disclosure is applied to the abdominal wall of a rat.
FIG. 3 shows images illustrating the test results of stability for formulations of Comparative Example 1, Comparative Example 2, and Examples 1 to 5. In Comparative Examples 1 and 2, the formulation was phase-separated, but in Examples 1 to 5, there was no change in the formulation.
FIG. 4 shows an image illustrating the test results of anti-adhesion efficacy for formulations of Control Examples 1 to 4 and Example 1 as a result of an autopsy.
FIG. 5 shows images representing the test results of anti-adhesion efficacy for formulations of Examples 6 to 21 as a result of an autopsy.

### [Mode for Disclosure]

Hereinafter, the present disclosure will be described in detail.

As used herein, the term "adhesion" may refer to a phenomenon in which skin, membranes, *etc.* that are separated from each other attach to each other. If tissue damage occurs after surgery or due to inflammation, foreign materials, bleeding, infection, wounds, friction, chemical treatment, *etc.,* blood leaks out and coagulates during the healing process of the wound, resulting in an adhesion phenomenon that causes abnormal bonding between surrounding tissues. These adhesions frequently occur, especially after surgery. It has been known that when an adhesion to the pelvis occurs, it may become the cause of chronic pain and sexual dysfunction; an adhesion after thyroidectomy may cause side effects such as chest pain and dysphagia; an adhesion after spinal surgery may cause compression of nerves thus causing severe pain; and an adhesion within the uterus may cause infertility, amenorrhea, and habitual miscarriage.

The anti-adhesion composition of the present disclosure may include a hydrophilic polymer at an appropriate concentration so that it can attach to the tissue surface without flowing down from the organ tissue even after sterilization. The hydrophilic polymer may be selected, for example, from the group consisting of starch, hyaluronic acid, sodium alginate, pectin, sodium carboxymethyl cellulose, methyl cellulose, hydroxypropyl methyl cellulose, low-substituted hydroxypropyl cellulose, hydroxypropyl cellulose, dextrin, dextran, pullulan, polyvinylpyrrolidone, polyacrylic acid, polyvinyl alcohol, polyethylene glycol, chitosan, gelatin, carrageenan, guar gum, locust bean gum, tragacanth, xanthan gum, gellan gum, agarose, and a combination thereof, but is not limited thereto. The hydrophilic polymers are those whose viscosity increases as the concentration increases, and preferably, the concentration of the hydrophilic polymer may be in an amount of 0.1 wt% to 20 wt% based on the total weight of the anti-adhesion composition. More preferably, the concentration of the hydrophilic polymer may be contained in an amount of 1 wt% to 10 wt% based on the total weight of the anti-adhesion composition. When the hydrophilic polymer is contained less than 0.1 wt%, the tissue adhesion becomes deteriorated due to low viscosity, whereas when the amount exceeds 20 wt%, it has a disadvantage in that it is impossible to prepare a uniform anti-adhesion agent due to too high viscosity.

The anti-adhesion composition of the present disclosure may include a suitable surfactant to form an emulsion. The surfactant may be selected, for example, from the group consisting of polyoxyethylene based fatty ethers derived from lauryl, cetyl, stearyl and oleyl alcohols, caprylocaproyl macrogol glycerides, polyethoxylated castor oil, ethyloxide/12-hydroxy stearic acid, sucrose fatty acid ester, a polyoxyethylene sorbitan fatty acid ester, sorbitan ester, a block copolymer of polyoxyethylene and polyoxypropylene, sodium lauryl sulfate, sodium oleate, sodium dioctyl sulfosuccinate, a quaternary ammonium salts an imidazoline salt, α-tocopherol-polyethylene glycol-1000-succinate, lecithin, and a combination thereof, but is not limited thereto. The surfactant may be included in a range of 0.1 wt% to 10 wt% based on the total weight of the anti-adhesion composition. Preferably, the surfactant may be included in a range of 0.1 wt% to 3 wt% based on the total weight of the anti-adhesion composition. When the surfactant is added in an amount of less than 0.1 wt%, an emulsion is not formed, whereas when its content exceeds 10 wt%, excessive bubbles are generated during the emulsification process, thus making it inappropriate for the manufacture.

The anti-adhesion composition of the present disclosure may include an appropriate co-surfactant (co-solvent) for formulation stability. The co-surfactant (co-solvent) may be selected, for example, from the group consisting of ethanol, glycerol, polyethylene glycol, propylene carbonate, propylene glycol, dimethylformamide, dimethyl sulfoxide, dimethylamine, triacetin, N-methyl-2-pyrrolidone, diethylene glycol monoethyl ether, polyoxylglyceride, and a combination thereof, but is not limited thereto. The co-surfactant (co-solvent) may be included in an amount of 1 wt% to 50 wt% based on the total weight of the anti-adhesion composition. Preferably, the co-surfactant (co-solvent) may be included in an amount of 15 wt% to 45 wt% based on the total weight of the total anti-adhesion composition. When the co-surfactant (co-solvent) is included less than 1 wt%, a stable formulation is not formed thus causing phase separation, whereas when its content exceeds 50 wt%, the flowability of the composition is excessively increased, thus making it inappropriate for the composition to attach to the tissue.

As the anti-adhesion agent of the present disclosure, oils derived from animals, plants, or minerals may be used. The oil is administered into the body in an oil phase and plays the role of maintaining slow absorption, and inhibits the occurrence of adhesions when applied to a wound site in the abdominal cavity. Examples of the oils with an anti-adhesion effect include soybean oil, canola oil, argan oil, *etc.* Suitable oils derived from animals, plants, or minerals may be selected, for example, from the group consisting of squalene, lanolin, mineral oil, paraffin, vaseline, ceresin, almond oil, corn oil, cottonseed oil, olive oil, peanut oil, safflower oil, grapeseed oil, sesame oil, soybean oil, argan oil, canola oil, castor oil, coconut oil, sunflower seed oil, palm oil, avocado oil, and a combination thereof, but is not limited thereto. The oil derived from animals, plants, or minerals may be included in a range of 1 wt% to 50 wt% based on the total weight of the anti-adhesion composition. Preferably, the oil derived from animals, plants or minerals may be included in a range of 10 wt% to 50 wt% based on the total weight of the anti-adhesion composition. When the oil derived from animals, plants, or minerals is included less than 1 wt%, an emulsion is not formed, whereas when its content exceeds 50 wt%, the resulting formulation is not stable thus causing a phenomenon of phase separation.

The anti-adhesion composition of the present disclosure may include a buffer solution. The buffer plays the role of maintaining the pH of the anti-adhesion agent from changing rapidly, and preferred examples of the buffer solution may include phosphate buffer, citrate buffer, histidine buffer, Tris buffer, *etc.,* but is not limited thereto.

The anti-adhesion composition of the present disclosure may include an isotonic agent. The isotonic agent reduces the difference in osmotic pressure between the body and the anti-adhesion agent when the anti-adhesion agent is administered into the body. Therefore, the isotonic agent plays the role of maintaining the osmotic pressure of the anti-adhesion agent to be suitable for administration into the body. Preferred examples of the isotonic agent may include polyvalent alcohols, amino acids, sodium chloride, potassium chloride, *etc.,* but is not limited thereto.

The anti-adhesion composition of the present disclosure may have an appropriate viscosity to enable the adhesion to the tissue surface without being flown down from the tissue of an organ. The anti-adhesion agent of the present disclosure may preferably have, at a shear rate of 1/s, a viscosity of 0 mPa s to 300,000 mPa s, more preferably 1,000 mPa s to 250,000 mPa·s, and an even more preferably 10,000 mPa·s to 200,000 mPa s. In addition, the anti-adhesion agent of the present disclosure is characterized in that it has a pseudoplastic flow, and thus the viscosity is temporarily lowered when applied to the wound site, thereby making it easy to apply the same to a wound site, and its viscosity is returned to a high viscosity again after application. Therefore, the anti-adhesion agent of the present disclosure has an advantage in that it does not leave the wound site. As a result, the anti-adhesion agent according to the present disclosure has advantages in that it not only provides an excellent anti-adhesion effect, but also enables an unskilled user to easily use the same.

The details of surgery to which the anti-adhesion agent of the present disclosure can be applied is not limited. The surgery to which the anti-adhesion agent of the present disclosure can be applied may be a surgery targeting the surface of a living organ. The part to which the anti-adhesion agent of the present disclosure is applicable may include a part where a wound is formed as a result of surgery, although it is not a direct target of surgery. When the anti-adhesion agent is brought into contact with the surface of an organ, the form of the anti-adhesion agent or the ratio of the active ingredients may be appropriately selected according to the type of organ and tissue, conditions and the area of the wound site, *etc.* The amount of contact of the anti-adhesion agent to the surface of a local organ may be to the extent that it covers the surface of the local organ.

The anti-adhesion agent of the present disclosure may be a formulation selected from the group including powders, granules, gels, sols, emulsions, dispersion systems, creams, solutions, and film formulations, but is not limited thereto, and it may be diversified according to the scope of medical application. For example, the anti-adhesion agent in the form of an emulsion may be prepared such that a co-surfactant and a hydrophilic polymer are dissolved in an aqueous phase, a surfactant is added thereto and stirred, and then the resulting mixture is uniformly mixed with an oil derived from animals, plants, or minerals under high pressure.

The anti-adhesion composition of the present disclosure is preferably in an emulsion formulation. Since the emulsion formulation has excellent biocompatibility and exhibits a behavior similar to a living tissue in a state when a large amount of water is absorbed, it can be widely applied in clinical practice regardless of surgical techniques.

Meanwhile, for the purpose of preventing adhesions from occurring after surgery, attempts have been made to prepare anti-adhesion agents by combining various living body-derived polymers and non-living body-derived polymers. However, when an anti-adhesion agent is prepared by mixing polymers known to have an anti-adhesion effect, the biocompatibility is excellent, but it has disadvantages in that the expected effect in tissue adhesion and duration in the body cannot be obtained; or alternatively, it may exhibit an anti-adhesion effect to some extent, but has low biocompatibility thus causing side effects. Therefore, it is difficult to develop an anti-adhesion agent which exhibits all of excellent biocompatibility, tissue adhesion, durability in the body, and particularly an excellent anti-adhesion effect by selecting an appropriate living body-derived polymer and a non-living body-derived polymer. Under the circumstances, the present inventors have endeavored to find a combination of materials that exhibits not only excellent biocompatibility, tissue adhesion, and durability in the body, but also ultimately exhibits an excellent anti-adhesion effect through the same; as a result, they have succeeded in developing an anti-adhesion agent including an oil derived from animals, plants, or minerals.

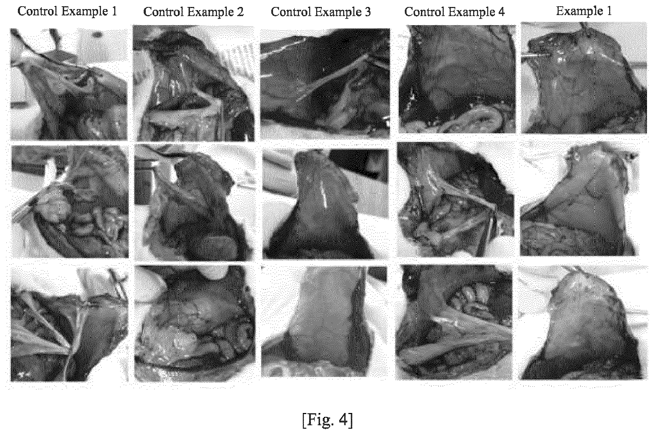

Specifically, Example 1 of the present disclosure discloses a composition in which sodium carboxymethyl cellulose, glycerol, polyoxyethylene sorbitan monopalmitate, and canola oil are mixed, and the anti-adhesion effect of the composition was confirmed compared to those of commercially available anti-adhesion agents (Control Example 2, Control Example) 3, and Control Example 4). As a result of the anti-adhesion animal test results by visual and histological observation at the time of autopsy, it was confirmed that the anti-adhesion agent of the present disclosure showed an excellent anti-adhesion effect in the degree of adhesion measured based on adhesion strength and adhesion area compared to the commercially available anti-adhesion agents (FIG. 4). Therefore, the anti-adhesion agent of the present disclosure can be effectively used for the purpose of preventing adhesions after surgery.

### Comparative Example 1

Sodium carboxymethyl cellulose (600 mg) was added to water (5,150 mg) and dissolved by stirring at 60°C for 3 hours. Then, polyoxyethylene sorbitan monopalmitate (250 mg) was added thereto and stirred for 2 hours to prepare a uniform solution. Canola oil (4,000 mg) was added to the solution and homogenized for 15 minutes under 13,500 rpm and 60°C conditions with a high pressure homogenizer to prepare a composition.

Table 1 shows the composition of Comparative Example 1.

**[Table 1]**

| Comparative Example 1 | | | |
|---|---|---|---|
| Type | Component | Weight (mg) | wt% |
| Hydrophilic Polymer | sodium carboxymethyl cellulose | 600 | 6 |
| Water Phase | water | 5150 | 51.5 |
| Surfactant | polyoxyethylene sorbitan monopalmitate | 250 | 2.5 |
| Oil Phase | canola oil | 4,000 | 40 |
| | Total Amount | 10,000 | 100 |

### Comparative Example 2

After mixing water (650 mg) and glycerol (3,000 mg), sodium carboxymethyl cellulose (600 mg) was added thereto and dissolved by stirring at 60°C for 3 hours. Polyoxyethylene sorbitan monopalmitate (250 mg) was added to the mixed solution and stirred for 2 hours to prepare a uniform solution. Canola oil (5,500 mg) was added to the solution and homogenized for 15 minutes under 13,500 rpm and 60°C conditions with a high pressure homogenizer to prepare a composition.

Table 2 shows the composition of Comparative Example 2.

**[Table 2]**

| Comparative Example 2 | | | |
|---|---|---|---|
| Type | Component | Weight (mg) | wt% |
| Hydrophilic Polymer | sodium carboxymethyl cellulose | 600 | 6 |
| Water Phase | water | 650 | 6.5 |
| Co-Surfactant (Co-Solvent) | glycerol | 3,000 | 30 |
| Surfactant | polyoxyethylene sorbitan monopalmitate | 250 | 2.5 |
| Oil Phase | canola oil | 5,500 | 55 |
| | Total Amount | 10,000 | 100 |

### Example 1

After mixing water (1,700 mg) and glycerol (3,450 mg), sodium carboxymethyl cellulose (600 mg) was added thereto and dissolved by stirring at 60°C for 3 hours. Polyoxyethylene sorbitan monopalmitate (250 mg) was added to the mixed solution and stirred for 2 hours to prepare a uniform solution. Canola oil (4,000 mg) was added to the solution and homogenized for 15 minutes under 13,500 rpm and 60°C conditions with a high pressure homogenizer to prepare a composition.

Table 3 shows the composition of Example 1.

**[Table 3]**

| Example 1 | | | |
|---|---|---|---|
| Type | Component | Weight (mg) | wt% |
| Hydrophilic Polymer | sodium carboxymethyl cellulose | 600 | 6 |
| Water Phase | water | 1,700 | 17 |
| Co-Surfactant (Co-Solvent) | glycerol | 3,450 | 34.5 |
| Surfactant | polyoxyethylene sorbitan monopalmitate | 250 | 2.5 |
| Oil Phase | canola oil | 4,000 | 40 |
| | Total Amount | 10,000 | 100 |

### Example 2

After mixing water (1,700 mg) and propylene glycol (3,450 mg), sodium carboxymethyl cellulose (600 mg) was added thereto and dissolved by stirring at 60°C for 3 hours. Polyoxyethylene sorbitan monopalmitate (250 mg) was added to the mixed solution and stirred for 2 hours to prepare a uniform solution. Canola oil (4,000 mg) was added to the solution and homogenized for 15 minutes under 13,500 rpm and 60°C conditions with a high pressure homogenizer to prepare a composition.

Table 4 shows the composition of Example 2.

**[Table 4]**

| Example 2 | | | |
|---|---|---|---|
| Type | Component | Weight (mg) | wt% |
| Hydrophilic Polymer | sodium carboxymethyl cellulose | 600 | 6 |
| Water Phase | water | 1,700 | 17 |
| Co-Surfactant (Co-Solvent) | propylene glycol | 3,450 | 34.5 |
| Surfactant | polyoxyethylene sorbitan monopalmitate | 250 | 2.5 |
| Oil Phase | canola oil | 4,000 | 40 |
| | Total Amount | 10,000 | 100 |

### Example 3

After mixing water (1,700 mg) and polyethylene glycol (PEG) 200 (3,450 mg), sodium carboxymethyl cellulose (600 mg) was added thereto and dissolved by stirring at 60°C for 3 hours. Polyoxyethylene sorbitan monopalmitate (250 mg) was added to the mixed solution and stirred for 2 hours to prepare a uniform solution. Canola oil (4,000 mg) was added to the solution and homogenized for 15 minutes under 13,500 rpm and 60°C conditions with a high pressure homogenizer to prepare a composition.

**[Table 5]**

| Example 3 | | | |
|---|---|---|---|
| Type | Component | Weight (mg) | wt% |
| Hydrophilic Polymer | sodium carboxymethyl cellulose | 600 | 6 |
| Water Phase | water | 1,700 | 17 |
| Co-Surfactant (Co-Solvent) | PEG 200 | 3,450 | 34.5 |
| Surfactant | polyoxyethylene sorbitan monopalmitate | 250 | 2.5 |
| Oil Phase | canola oil | 4,000 | 40 |
| | Total Amount | 10,000 | 100 |

### Example 4

After mixing water (1,700 mg) and PEG 400 (3,450 mg), sodium carboxymethyl cellulose (600 mg) was added thereto and dissolved by stirring at 60°C for 3 hours. Polyoxyethylene sorbitan monopalmitate (250 mg) was added to the mixed solution and stirred for 2 hours to prepare a uniform solution. Canola oil (4,000 mg) was added to the solution and homogenized for 15 minutes under 13,500 rpm and 60°C conditions with a high pressure homogenizer to prepare a composition.

Table 6 shows the composition of Example 4.

**[Table 6]**

| Example 4 | | | |
|---|---|---|---|
| Type | Component | Weight (mg) | wt% |
| Hydrophilic Polymer | sodium carboxymethyl cellulose | 600 | 6 |
| Water Phase | water | 1,700 | 17 |
| Co-Surfactant (Co-Solvent) | PEG 400 | 3,450 | 34.5 |
| Surfactant | polyoxyethylene sorbitan monopalmitate | 250 | 2.5 |
| Oil Phase | canola oil | 4,000 | 40 |
| | Total Amount | 10,000 | 100 |

### Example 5

After mixing water (1,700 mg) and diethylene glycol monoethyl ether (3,450 mg), sodium carboxymethyl cellulose (600 mg) was added thereto and dissolved by stirring at 60°C for 3 hours. Polyoxyethylene sorbitan monopalmitate (250 mg) was added to the mixed solution and stirred for 2 hours to prepare a uniform solution. Canola oil (4,000 mg) was added to the solution and homogenized for 15 minutes under 13,500 rpm and 60°C conditions with a high pressure homogenizer to prepare a composition.

Table 7 shows the composition of Example 5.

**[Table 7]**

| Example 5 | | | |
|---|---|---|---|
| Type | Component | Weight (mg) | wt% |
| Hydrophilic Polymer | sodium carboxymethyl cellulose | 600 | 6 |
| Water Phase | water | 1,700 | 17 |
| Co-Surfactant (Co-Solvent) | diethylene glycol monoethyl ether | 3,450 | 34.5 |
| Surfactant | polyoxyethylene sorbitan monopalmitate | 250 | 2.5 |
| Oil Phase | canola oil | 4,000 | 40 |
| | Total Amount | 10,000 | 100 |

### Example 6

After mixing water (1,700 mg) and glycerol (3,450 mg), sodium carboxymethyl cellulose (600 mg) was added thereto and dissolved by stirring at 60°C for 3 hours. Polyoxyethylene sorbitan monopalmitate (250 mg) was added to the mixed solution and stirred for 2 hours to prepare a uniform solution. Canola oil (4,000 mg) was added to the solution and homogenized for 15 minutes under 13,500 rpm and 60°C conditions with a high pressure homogenizer to prepare a composition.

Table 8 shows the composition of Example 6.

**[Table 8]**

| Example 6 | | | |
|---|---|---|---|
| Type | Component | Weight (mg) | wt% |
| Hydrophilic Polymer | sodium carboxymethyl cellulose | 600 | 6 |
| Water Phase | water | 1,700 | 17 |
| Co-Surfactant (Co-Solvent) | glycerol | 3,450 | 34.5 |
| Surfactant | polyoxyethylene sorbitan monopalmitate | 250 | 2.5 |
| Oil Phase | canola oil | 4,000 | 40 |
| | Total Amount | 10,000 | 100 |

### Example 7

After mixing water (1,700 mg) and glycerol (3,450 mg), sodium carboxymethyl cellulose (600 mg) was added thereto and dissolved by stirring at 60°C for 3 hours. Polyoxyethylene sorbitan monopalmitate (250 mg) was added to the mixed solution and stirred for 2 hours to prepare a uniform solution. Cottonseed oil (4,000 mg) was added to the solution and homogenized for 15 minutes under 13,500 rpm and 60°C conditions with a high pressure homogenizer to prepare a composition.

Table 9 shows the composition of Example 7.

**[Table 9]**

| Example 7 | | | |
|---|---|---|---|
| Type | Component | Weight (mg) | wt% |
| Hydrophilic Polymer | sodium carboxymethyl cellulose | 600 | 6 |
| Water Phase | water | 1,700 | 17 |
| Co-Surfactant (Co-Solvent) | glycerol | 3,450 | 34.5 |
| Surfactant | polyoxyethylene sorbitan monopalmitate | 250 | 2.5 |
| Oil Phase | cottonseed oil | 4,000 | 40 |
| | Total Amount | 10,000 | 100 |

### Example 8

After mixing water (1,700 mg) and glycerol (3,450 mg), sodium carboxymethyl cellulose (600 mg) was added thereto and dissolved by stirring at 60°C for 3 hours. Polyoxyethylene sorbitan monopalmitate (250 mg) was added to the mixed solution and stirred for 2 hours to prepare a uniform solution. Olive oil (4,000 mg) was added to the solution and homogenized for 15 minutes under 13,500 rpm and 60°C conditions with a high pressure homogenizer to prepare a composition.

Table 10 shows the composition of Example 8.

**[Table 10]**

| Example 8 | | | |
|---|---|---|---|
| Type | Component | Weight (mg) | wt% |
| Hydrophilic Polymer | sodium carboxymethyl cellulose | 600 | 6 |
| Water Phase | water | 1,700 | 17 |
| Co-Surfactant (Co-Solvent) | glycerol | 3,450 | 34.5 |
| Surfactant | polyoxyethylene sorbitan monopalmitate | 250 | 2.5 |
| Oil Phase | olive oil | 4,000 | 40 |
| | Total Amount | 10,000 | 100 |

### Example 9

After mixing water (1,700 mg) and glycerol (3,450 mg), sodium carboxymethyl cellulose (600 mg) was added thereto and dissolved by stirring at 60°C for 3 hours. Polyoxyethylene sorbitan monopalmitate (250 mg) was added to the mixed solution and stirred for 2 hours to prepare a uniform solution. Soybean oil (4,000 mg) was added to the solution and homogenized for 15 minutes under 13,500 rpm and 60°C conditions with a high pressure homogenizer to prepare a composition.

Table 11 shows the composition of Example 9.

**[Table 11]**

| Example 9 | | | |
|---|---|---|---|
| Type | Component | Weight (mg) | wt% |
| Hydrophilic Polymer | sodium carboxymethyl cellulose | 600 | 6 |
| Water Phase | water | 1,700 | 17 |
| Co-Surfactant (Co-Solvent) | glycerol | 3,450 | 34.5 |
| Surfactant | polyoxyethylene sorbitan monopalmitate | 250 | 2.5 |
| Oil Phase | soybean oil | 4,000 | 40 |
| | Total Amount | 10,000 | 100 |

### Example 10

After mixing water (1,700 mg) and glycerol (3,450 mg), sodium carboxymethyl cellulose (600 mg) was added thereto and dissolved by stirring at 60°C for 3 hours. Polyoxyethylene sorbitan monopalmitate (250 mg) was added to the mixed solution and stirred for 2 hours to prepare a uniform solution. Castor oil (4,000 mg) was added to the solution and homogenized for 15 minutes under 13,500 rpm and 60°C conditions with a high pressure homogenizer to prepare a composition.

Table 12 shows the composition of Example 10.

**[Table 12]**

| Example 10 | | | |
|---|---|---|---|
| Type | Component | Weight (mg) | wt% |
| Hydrophilic Polymer | sodium carboxymethyl cellulose | 600 | 6 |
| Water Phase | water | 1,700 | 17 |
| Co-Surfactant (Co-Solvent) | glycerol | 3,450 | 34.5 |
| Surfactant | polyoxyethylene sorbitan monopalmitate | 250 | 2.5 |
| Oil Phase | castor oil | 4,000 | 40 |
| | Total Amount | 10,000 | 100 |

### Example 11

After mixing water (1,700 mg) and glycerol (3,450 mg), sodium carboxymethyl cellulose (600 mg) was added thereto and dissolved by stirring at 60°C for 3 hours. Polyoxyethylene sorbitan monopalmitate (250 mg) was added to the mixed solution and stirred for 2 hours to prepare a uniform solution. Squalene (4,000 mg) was added to the solution and homogenized for 15 minutes under 13,500 rpm and 60°C conditions with a high pressure homogenizer to prepare a composition.

Table 13 shows the composition of Example 11.

**[Table 13]**

| Example 11 | | | |
|---|---|---|---|
| Type | Component | Weight (mg) | wt% |
| Hydrophilic Polymer | sodium carboxymethyl cellulose | 600 | 6 |
| Water Phase | water | 1,700 | 17 |
| Co-Surfactant (Co-Solvent) | glycerol | 3,450 | 34.5 |
| Surfactant | polyoxyethylene sorbitan monopalmitate | 250 | 2.5 |
| Oil Phase | squalene | 4,000 | 40 |
| | Total Amount | 10,000 | 100 |

### Example 12

After mixing water (1,700 mg) and glycerol (3,450 mg), hyaluronic acid (600 mg) was added thereto and dissolved by stirring at 60°C for 3 hours. Polyoxyethylene sorbitan monopalmitate (250 mg) was added to the mixed solution and stirred for 2 hours to prepare a uniform solution. Canola oil (4,000 mg) was added to the solution and homogenized for 15 minutes under 13,500 rpm and 60°C conditions with a high pressure homogenizer to prepare a composition.

Table 14 shows the composition of Example 12.

**[Table 14]**

| Example 12 | | | |
|---|---|---|---|
| Type | Component | Weight (mg) | wt% |
| Hydrophilic Polymer | sodium carboxymethyl cellulose | 600 | 6 |
| Water Phase | water | 1,700 | 17 |
| Co-Surfactant (Co-Solvent) | glycerol | 3,450 | 34.5 |
| Surfactant | polyoxyethylene sorbitan monopalmitate | 250 | 2.5 |
| Oil Phase | canola oil | 4,000 | 40 |
| | Total Amount | 10,000 | 100 |

### Example 13

After mixing water (1,700 mg) and glycerol (3,450 mg), PEG 20000 (600 mg) was added thereto and dissolved by stirring at 60°C for 3 hours. Polyoxyethylene sorbitan monopalmitate (250 mg) was added to the mixed solution and stirred for 2 hours to prepare a uniform solution. Canola oil (4,000 mg) was added to the solution and homogenized for 15 minutes under 13,500 rpm and 60°C conditions with a high pressure homogenizer to prepare a composition.

Table 15 shows the composition of Example 13.

**[Table 15]**

| Example 13 | | | |
|---|---|---|---|
| Type | Component | Weight (mg) | wt% |
| Hydrophilic Polymer | PEG 20000 | 600 | 6 |
| Water Phase | water | 1,700 | 17 |
| Co-Surfactant (Co-Solvent) | glycerol | 3,450 | 34.5 |
| Surfactant | polyoxyethylene sorbitan monopalmitate | 250 | 2.5 |
| Oil Phase | canola oil | 4,000 | 40 |
| | Total Amount | 10,000 | 100 |

### Example 14

After mixing water (1,700 mg) and glycerol (3,450 mg), carrageenan (600 mg) was added thereto and dissolved by stirring at 60°C for 3 hours. Polyoxyethylene sorbitan monopalmitate (250 mg) was added to the mixed solution and stirred for 2 hours to prepare a uniform solution. Canola oil (4,000 mg) was added to the solution and homogenized for 15 minutes under 13,500 rpm and 60°C conditions with a high pressure homogenizer to prepare a composition.

Table 16 shows the composition of Example 14.

**[Table 16]**

| Example 14 | | | |
|---|---|---|---|
| Type | Component | Weight (mg) | wt% |
| Hydrophilic Polymer | carrageenan | 600 | 6 |
| Water Phase | water | 1,700 | 17 |
| Co-Surfactant (Co-Solvent) | glycerol | 3,450 | 34.5 |
| Surfactant | polyoxyethylene sorbitan monopalmitate | 250 | 2.5 |
| Oil Phase | canola oil | 4,000 | 40 |
| | Total Amount | 10,000 | 100 |

### Example 15

After mixing water (1,700 mg) and glycerol (3,450 mg), starch (600 mg) was added thereto and dissolved by stirring at 60°C for 3 hours. Polyoxyethylene sorbitan monopalmitate (250 mg) was added to the mixed solution and stirred for 2 hours to prepare a uniform solution. Canola oil (4,000 mg) was added to the solution and homogenized for 15 minutes under 13,500 rpm and 60°C conditions with a high pressure homogenizer to prepare a composition.

Table 17 shows the composition of Example 15.

**[Table 17]**

| Example 15 | | | |
|---|---|---|---|
| Type | Component | Weight (mg) | wt% |
| Hydrophilic Polymer | starch | 600 | 6 |
| Water Phase | water | 1,700 | 17 |
| Co-Surfactant (Co-Solvent) | glycerol | 3,450 | 34.5 |
| Surfactant | polyoxyethylene sorbitan monopalmitate | 250 | 2.5 |
| Oil Phase | canola oil | 4,000 | 40 |
| | Total Amount | 10,000 | 100 |

### Example 16

After mixing water (1,700 mg) and glycerol (3,450 mg), sodium alginate (600 mg) was added thereto and dissolved by stirring at 60°C for 3 hours. Polyoxyethylene sorbitan monopalmitate (250 mg) was added to the mixed solution and stirred for 2 hours to prepare a uniform solution. Canola oil (4,000 mg) was added to the solution and homogenized for 15 minutes under 13,500 rpm and 60°C conditions with a high pressure homogenizer to prepare a composition.

Table 18 shows the composition of Example 16.

**[Table 18]**

| Example 16 | | | |
|---|---|---|---|
| Type | Component | Weight (mg) | wt% |
| Hydrophilic Polymer | sodium alginate | 600 | 6 |
| Water Phase | water | 1,700 | 17 |
| Co-Surfactant (Co-Solvent) | glycerol | 3,450 | 34.5 |
| Surfactant | polyoxyethylene sorbitan monopalmitate | 250 | 2.5 |
| Oil Phase | canola oil | 4,000 | 40 |
| | Total Amount | 10,000 | 100 |

### Example 17

After mixing water (1,700 mg) and glycerol (3,450 mg), gelatin (600 mg) was added thereto and dissolved by stirring at 60°C for 3 hours. Polyoxyethylene sorbitan monopalmitate (250 mg) was added to the mixed solution and stirred for 2 hours to prepare a uniform solution. Canola oil (4,000 mg) was added to the solution and homogenized for 15 minutes under 13,500 rpm and 60°C conditions with a high pressure homogenizer to prepare a composition.

Table 19 shows the composition of Example 17.

**[Table 19]**

| Example 17 | | | |
|---|---|---|---|
| Type | Component | Weight (mg) | wt% |
| Hydrophilic Polymer | gelatin | 600 | 6 |
| Water Phase | water | 1,700 | 17 |
| Co-Surfactant (Co-Solvent) | glycerol | 3,450 | 34.5 |
| Surfactant | polyoxyethylene sorbitan monopalmitate | 250 | 2.5 |
| Oil Phase | canola oil | 4,000 | 40 |
| | Total Amount | 10,000 | 100 |

### Example 18

After mixing water (1,700 mg) and glycerol (3,450 mg), chitosan (600 mg) was added thereto and dissolved by stirring at 60°C for 3 hours. Polyoxyethylene sorbitan monopalmitate (250 mg) was added to the mixed solution and stirred for 2 hours to prepare a uniform solution. Canola oil (4,000 mg) was added to the solution and homogenized for 15 minutes under 13,500 rpm and 60°C conditions with a high pressure homogenizer to prepare a composition.

Table 20 shows the composition of Example 18.

**[Table 20]**

| Example 18 | | | |
|---|---|---|---|
| Type | Component | Weight (mg) | wt% |
| Hydrophilic Polymer | chitosan | 600 | 6 |
| Water Phase | water | 1,700 | 17 |
| Co-Surfactant (Co-Solvent) | glycerol | 3,450 | 34.5 |
| Surfactant | polyoxyethylene sorbitan monopalmitate | 250 | 2.5 |
| Oil Phase | canola oil | 4,000 | 40 |
| | Total Amount | 10,000 | 100 |

### Example 19

After mixing water (1,700 mg) and glycerol (3,450 mg), pectin (600 mg) was added thereto and dissolved by stirring at 60°C for 3 hours. Polyoxyethylene sorbitan monopalmitate (250 mg) was added to the mixed solution and stirred for 2 hours to prepare a uniform solution. Canola oil (4,000 mg) was added to the solution and homogenized for 15 minutes under 13,500 rpm and 60°C conditions with a high pressure homogenizer to prepare a composition.

Table 21 shows the composition of Example 19.

**[Table 21]**

| Example 19 | | | |
|---|---|---|---|
| Type | Component | Weight (mg) | wt% |
| Hydrophilic Polymer | pectin | 600 | 6 |
| Water Phase | water | 1,700 | 17 |
| Co-Surfactant (Co-Solvent) | glycerol | 3,450 | 34.5 |
| Surfactant | polyoxyethylene sorbitan monopalmitate | 250 | 2.5 |
| Oil Phase | canola oil | 4,000 | 40 |
| | Total Amount | 10,000 | 100 |

### Example 20

After mixing water (1,700 mg) and glycerol (3,450 mg), xanthan gum (600 mg) was added thereto and dissolved by stirring at 60°C for 3 hours. Polyoxyethylene sorbitan monopalmitate (250 mg) was added to the mixed solution and stirred for 2 hours to prepare a uniform solution. Canola oil (4,000 mg) was added to the solution and homogenized for 15 minutes under 13,500 rpm and 60°C conditions with a high pressure homogenizer to prepare a composition.

Table 22 shows the composition of Example 20.

**[Table 22]**

| Example 20 | | | |
|---|---|---|---|
| Type | Component | Weight (mg) | wt% |
| Hydrophilic Polymer | xanthan gum | 600 | 6 |
| Water Phase | water | 1,700 | 17 |
| Co-Surfactant (Co-Solvent) | glycerol | 3,450 | 34.5 |
| Surfactant | polyoxyethylene sorbitan monopalmitate | 250 | 2.5 |
| Oil Phase | canola oil | 4,000 | 40 |
| | Total Amount | 10,000 | 100 |

### Example 21

After mixing water (1,700 mg) and glycerol (3,450 mg), agarose (600 mg) was added thereto and dissolved by stirring at 60°C for 3 hours. Polyoxyethylene sorbitan monopalmitate (250 mg) was added to the mixed solution and stirred for 2 hours to prepare a uniform solution. Canola oil (4,000 mg) was added to the solution and homogenized for 15 minutes under 13,500 rpm and 60°C conditions with a high pressure homogenizer to prepare a composition.

Table 23 shows the composition of Example 21.

**[Table 23]**

| Example 21 | | | |
|---|---|---|---|
| Type | Component | Weight (mg) | wt% |
| Hydrophilic Polymer | xanthan gum | 600 | 6 |
| Water Phase | water | 1,700 | 17 |
| Co-Surfactant (Co-Solvent) | glycerol | 3,450 | 34.5 |
| Surfactant | polyoxyethylene sorbitan monopalmitate | 250 | 2.5 |
| Oil Phase | canola oil | 4,000 | 40 |
| | Total Amount | 10,000 | 100 |

### Control Example 1: Physiological saline

A negative control group, in which the friction injury site was treated with physiological saline, was established.

### Control Example 2: Guardix^{®}-sol (Hanmi Pharm Co., Ltd.)

As an anti-adhesion agent consisting of hyaluronic acid and carboxymethyl cellulose, Guardix-sol (Hanmi Pharm Co., Ltd.), a commercially available product, was used.

### Control Example 3: Guardix^{®}-SG (Hanmi Pharm Co., Ltd.)

As an anti-adhesion agent using alginate, calcium chloride, and poloxamer (a temperature-sensitive polymer), Guardix^{®}-SG, which is a commercially available anti-adhesion agent having a characteristic in that it is in a sol state at low temperatures and becomes a gel at room temperature or higher, was used.

### Control Example 4: Medicurtain^{®} (Shin Poong Pharm. Co., Ltd.)

This control example is characterized in that as an anti-adhesion agent consisting of hyaluronic acid and hydroxyethyl starch, hydroxyethyl starch that has anti-inflammatory and anti-blood clotting properties was used. Medicurtain^{®} (Shin Poong Pharm. Co., Ltd.), which is commercially available anti-adhesion agent, was used.

### Experimental Example 1: Formulation stability test

The formulation stability was tested by preparing formulations of Comparative Example 1, Comparative Example 2, and Examples 1 to 5 of the present disclosure, and the method is as follows. 1 g of each of the prepared formulation was weighed and added into an Eppendorf tube (1.5 mL). The tubes containing each formulation were frozen at -20°C for 20 hours and thawed by storing at 60°C for 4 hours. After repeating the freezing (-20 °C) and thawing (60 °C) process three times, the tubes were subjected to centrifugation for 15 minutes at 5,000 rpm with a centrifuge. Then, the stability of each formulation was visually determined.

The results of this test are shown in FIG. 3. The results showed that phase separation occurred in the formulations of Comparative Examples 1 and 2, whereas the formulations of Examples 1 to 5 remained stable. As a result, it can be confirmed that a stable phase could not be formed in formulations where a co-surfactant was not used as in Comparative Example 1 or where an excessive amount of oil was included as in Comparative Example 2.

### Experimental Example 2: Animal test of anti-adhesion efficacy of anti-adhesion agent - visual findings at autopsy

An experiment was conducted to determine the anti-adhesion efficacy of anti-adhesion agents through observation of macroscopic findings during autopsy. After anesthesia, the hair on the surgical site of experiment animals was removed, disinfected with povidone, and an incision was made about 4 cm to 5 cm along the midline of the abdominal cavity. The adhesion between the abdominal wall and tissue was induced by generating an abrasion with a size of 1.5 cm × 1.5 cm on the peritoneal membrane using sandpaper. 0.5 mL of each test substance was injected thereinto. Animals that survived until the end of the test period were euthanized using the CO₂ gas inhalation method, and gross pathological examination was performed by autopsy, and the degree of adhesion was evaluated based on the adhesion strength and adhesion area for each individual. The negative control group was set as Control Example 1, in which the site where the adhesion was induced was treated with physiological saline, whereas the positive control group was set as Control Examples 2 to 4 in which the site where the adhesion was induced was treated with commercially available anti-adhesion agents, and thereby, the anti-adhesion efficacy of the anti-adhesion agents of Example 1 and Examples 6 to 21, which are the examples of the present disclosure, was evaluated. (Table 25).

The evaluation of adhesions by observation of visual findings was performed by the methods of Ntourakis, Dimitrios, *et al.* (Ntourakis, Dimitrios, Michail Katsimpoulas, Anna Tanoglidi, Calypso Barbatis, Panayotis E Karayannacos, Theodoros N Sergentanis, Nikolaos Kostomitsopoulos, and Anastasios Machairas. "Adhesions and Healing of Intestinal Anastomoses: The Effect of Anti-Adhesion Barriers." Surgical Innovation 23, No. 3 (2016): 266-76.)). Specifically, the degree of adhesion was evaluated by classification (0 to 4, Table 24) according to the size, thickness, and force required to separate the adhesions on the adhesion surface, and the results are shown in Table 25.

**[Table 24]**

| Score | Description |
|---|---|
| 0 | formation of no adhesion |
| 1 | formation of thin, film-like adhesion and easy separation without much strength |
| 2 | thick and a little strength is required to separate the adhesion to be pulled taut |
| 3 | thick and it is impossible to separate adhesion without scissors (i.e., it is impossible to separate adhesion by the pulling force alone) |
| 4 | formation of adhesion by intermingling with organs in the abdominal cavity |

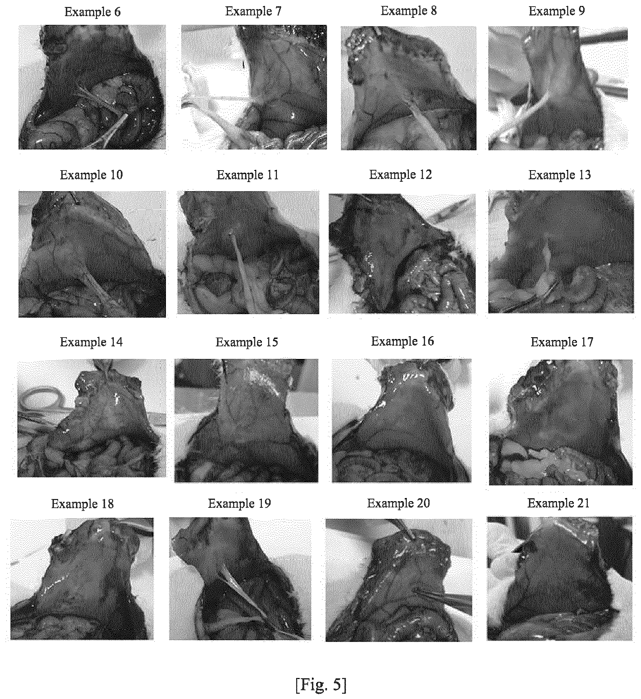

As shown in FIG. 4, it was confirmed that the anti-adhesion agent of the present disclosure (Example 1), in which carboxymethylcellulose sodium, glycerol, and canola oil are mixed, could prevent adhesions more effectively in terms of the degree of adhesion, adhesion strength, adhesion area, *etc.,* compared to the group treated with physiological saline (Control Example 1), Guardix^{®}-sol (Hanmi Pharm Co., Ltd.) (Control Example 2), Guardix^{®}-SG (Hanmi Pharm Co., Ltd.) (Control Example 3), and Medicurton^{®} (Shin Poong Pharm. Co., Ltd.) (Control Example 4) (FIG. 4). The anti-adhesion effect according to the type of oil phase was confirmed through Examples 6 to 11 of the present disclosure. In addition, it was confirmed that the anti-adhesion agents of Examples 12 to 21 of the present disclosure have an excellent anti-adhesion effect (FIG. 5).

**[Table 25]**

| Test Group | No. 1 | No. 2 | No. 3 | Mean Score of Adhesion Evaluation (n = 3) |
|---|---|---|---|---|
| Control Example 1 | 4 | 3 | 3 | 3.67 |
| Control Example 2 | 2 | 3 | 1 | 2 |
| Control Example 3 | 1 | 0 | 0 | 0.33 |
| Control Example 4 | 0 | 2 | 3 | 1.67 |
| Example 1 | 0 | 0 | 0 | 0 |
| Example 6 | 0 | 1 | 0 | 0.33 |
| Example 7 | 1 | 1 | 0 | 0.67 |
| Example 8 | 1 | 0 | 0 | 0.33 |
| Example 9 | 0 | 0 | 1 | 0.33 |
| Example 10 | 0 | 1 | 2 | 1 |
| Example 11 | 1 | 1 | 1 | 1 |
| Example 12 | 0 | 0 | 0 | 0 |
| Example 13 | 0 | 1 | 0 | 0.33 |
| Example 14 | 0 | 0 | 0 | 0 |
| Example 15 | 0 | 0 | 0 | 0 |
| Example 16 | 0 | 0 | 0 | 0 |
| Example 17 | 0 | 0 | 0 | 0 |
| Example 18 | 0 | 0 | 0 | 0 |
| Example 19 | 1 | 0 | 0 | 0.33 |
| Example 20 | 0 | 0 | 0 | 0 |
| Example 21 | 0 | 0 | 0 | 0 |

## Claims

1. An anti-adhesion composition comprising:
a hydrophilic polymer;
an oil derived from animals, plants, or minerals;
a surfactant; and
a co-surfactant selected from the group consisting of ethanol, glycerol, polyethylene glycol, propylene carbonate, propylene glycol, dimethylformamide, dimethyl sulfoxide, dimethylamine, triacetin, N-methyl-2-pyrrolidone, diethylene glycol monoethyl ether, polyoxyl glycerides, and a combination thereof,
wherein the co-surfactant is contained in a range of 15 wt% to 45 wt% based on the total weight; and
wherein the oil derived from animals, plants or minerals is contained in a range of 10 wt% to 50 wt% based on the total weight.

2. The anti-adhesion composition of claim 1, wherein the hydrophilic polymer is contained in a range of 0. 1 wt% to 20 wt% based on the total weight.

3. The anti-adhesion composition of claim 1, wherein the surfactant is contained in a range of 0. 1 wt% to 10 wt% based on the total weight.

4. The anti-adhesion composition of claim 1, wherein the viscosity of the anti-adhesion composition is in a range of 0 mPa·s to 300,000 mPa·s.

5. The anti-adhesion composition of claim 1, wherein the hydrophilic polymer is selected from the group consisting of starch, hyaluronic acid, sodium alginate, pectin, carboxymethyl cellulose sodium, methyl cellulose, hydroxypropyl methyl cellulose, low-substituted hydroxypropyl cellulose, hydroxypropyl cellulose, dextran, pullulan, polyvinylpyrrolidone, polyacrylic acid, polyvinyl alcohol, polyethylene glycol, chitosan, gelatin, carrageenan, guar gum, locust bean gum, tragacantha, xanthan gum, gellan gum, agarose, and a combination thereof.

6. The anti-adhesion composition of claim 1, wherein the oil derived from animals, plants, or minerals is selected from the group consisting of squalene, lanolin, mineral oil, paraffin, vaseline, almond oil, corn oil, cottonseed oil, olive oil, peanut oil, safflower oil, grapeseed oil, sesame oil, soybean oil, argan oil, canola oil, castor oil, coconut oil, sunflower oil, palm oil, avocado oil, and a combination thereof.

7. The anti-adhesion composition of claim 1, wherein the surfactant is selected from the group consisting of polyoxyethylene based fatty ethers derived from lauryl, cetyl, stearyl, and oleyl alcohols, caprylocaproyl macrogol glycerides, polyethoxylated castor oil, ethyloxide/12-hydroxy stearic acid, sucrose fatty acid ester, polyoxyethylene sorbitan fatty acid ester, sorbitan ester, a block copolymer of polyoxyethylene and polyoxypropylene, sodium lauryl sulfate, sodium oleate, sodium dioctyl sulfosuccinate, quaternary ammonium salt, imidazoline salt, α-tocopherol-polyethylene glycol-1000-succinate, lecithin, and a combination thereof.
